Europäisches Patentamt

European Patent Office

Office européen des brevets

(19)

(11) Publication number: **0 424 159 A2**

# EUROPEAN PATENT APPLICATION

(12)

(21) Application number: 90311464.3

(22) Date of filing: 18.10.90

(51) Int. Cl.⁵: **A61L 2/00, A61L 27/00**

(30) Priority: **19.10.89 US 424339**

(43) Date of publication of application:
**24.04.91 Bulletin 91/17**

(84) Designated Contracting States:
**BE DE FR GB IT NL**

(71) Applicant: OSTEOTECH, INC.,
1151E Shrewsbury Avenue
Shrewsbury New Jersey 07701(US)

(72) Inventor: Callen, John P.
88 Windsor Drive
Eatontown, New Jersey 07724(US)
Inventor: O'Leary, Robert K.
110 St. Clair Avenue
Spring Lake, New Jersey 07762(US)

(74) Representative: Marsh, Roy David et al
Urquhart-Dykes & Lord Midsummer House
411C Midsummer Boulevard
Central Milton Keynes MK9 3BN(GB)

(54) Aseptic processing of allograft bone and tissue.

(57) The present invention comprises a method for processing allograft bone or tissue, said method comprising the steps of:

(i) subjecting allograft bone or tissue to sterilization, said sterilization sufficient to kill microorganisms but insufficient to cause substantial physical, chemical or biological deterioration of the allograft bone or tissue; and

(ii) aseptically processing the sterilized allograft bone or tissue resulting from step (i).

EP 0 424 159 A2

Xerox Copy Centre

## ASEPTIC PROCESSING OF ALLOGRAFT BONE AND TISSUE

This invention relates to allograft bone and tissue and more particularly to the aseptic processing of allograft bone and musculoskeletal tissue prior to the implantation.

Recently, the transplantation of allograft bone and tissue has become a relatively widely utilized medical procedure. Bone and tissue allografts are used to repair or replace defective or damaged bones, tendons, ligaments and the like. Thus, the range of allograft transplants includes entire joints, sections of long bone, bone chips for surgical procedures such as spinal fusion and craniotomies, bone dust used in dental repair and/or reconstruction and musculoskeletal soft tissue such as ligaments and tendons.

The biological and biomechanical quality of allograft tissue depends largely on the retrieval, preservation and storage methods employed. Bone or tissue may be recovered using sterile technique in a sterile environment or a nonsterile area requiring secondary (final) sterilization of tissue prior to transplantation. The major advantages of sterile recovery include (1) the bone or tissue retains its natural sterility and (2) there is no need for secondary final product sterilization that may alter biological or biomechanical properties or leave undesirable residues.

The most frequently used methods of secondary final product sterilization include exposure to high-dose irradiation or high dose (high concentration) ethylene oxide. High-dose irradiation and high dose ethylene oxide, i.e. high dose toxic sterilization techniques, however, can infringe upon the bone or tissues' biologic properties and also can disrupt collagen cross-linkage that is important to mechanical strength.

Accordingly, methods for processing allograft bone and tissue which provide surgically sterile products and which do not interfere with the natural integrity or bioactivity, i.e. do not interfere with the osteogenic properties, of the allograft bone or tissue are highly desired.

Therefore, it is one object of the present invention to provide a novel method for processing allograft bone or musculoskeletal tissue.

Another object of this invention is to provide a novel method for processing allograft bone or tissue which avoids the detrimental effects of high dose toxic sterilization techniques and which provides sterile products having maximum structural integrity, biomechanical properties and bioactivity.

These and other objects are achieved herein by an aseptic method for processing allograft bone or tissue comprising the steps of:

(i) subjecting allograft bone or tissue to sterilization, said sterilization being sufficient to kill microorganisms, but insufficient to cause substantial physical chemical or biological deterioration of the allograft bone or tissue followed by:

(ii) aseptic cleansing of the allograft bone or tissue resulting from step (i).

Bone and musculoskeletal tissue to be processed in accordance with the present invention are procured, typically by trained personnel, under surgically sterile conditions, from donor cadavers. Once procured, the bone and tissue is ready to be processed in accordance with the aseptic process of the present invention.

Thus, in accordance with the present invention, the allograft bone or tissue is subjected to a sterilization procedure prior to further aseptic processing of the same. Sterilization procedures contemplated by the present invention include any such procedure which is capable of killing microorganisms, but which does not substantially interfere with the physical, chemical and biological properties, such as the osteogenic properties, of the bony tissue. Included within the definition of microorganisms intended to be killed by the sterilization step of the present process are all vegetative and spore forming microorganisms including viruses. Typical sterilization procedures encompassed by the present invention include irradiation, such as gamma radiation, electron beam radiation, ultraviolet radiation and the like, chemical sterilization such as ethylene oxide, formaldehyde, vapor phase hydrogen peroxide, chlorine dioxide, ethanol, ozone, treatment with surface active agents, such as quaternary agents, phenolic agents, glutaraldehyde and the like and thermal procedures such as ultrapasteurization.

The preferred sterilization procedure for the purposes of the present invention is low dose irradiation and particularly low doses of gamma radiation. Thus, in accordance with the preferred method of the present invention, treatment of the allograft bone or tissue comprises exposure of the allograft material to gamma radiation, such as from a cobalt-60 source or a cesium-137 source, at a dosage level sufficient to kill microorganisms but insufficient to cause substantial deterioration of the bone. For the purposes of the present invention such dose levels are generally within the range of from about 0.1 to about 2 megarads and, preferably about 1.0 to about 1.6 megarads.

Since absolute sterilization is impossible to attain, the accepted standard of sterility is defined in terms of probability of survival wherein the probability relates to the probable number of test units containing one viable organism. The usually accepted definition of secondary or terminal sterility is one viable microorganism per million test units. However, with the aseptic process of the present invention one microorganism per thousand test units is acceptable. Therefore, the effective amount of gamma radiation for allograft bone can range, for example from about 0.1 megarads to a maximum dose below that level which would cause substantial chemical, physical or biological deterioration to the allograft bone, i.e. about 2 megarads. Thus, in accordance with the present process, for example, with a bioburden of 100 microorganisms per test unit, the amount of gamma radiation necessary to achieve the one per thousand survival is about 1.0 megarads, and the amount of gamma radiation necessary to achieve the one per million survival rate is about 1.6 megarads.

As stated above, other irradiation sterilization procedures contemplated within the scope of the present invention include electron beam (E-beam) irradiation. Such a procedure would involve, for example, exposing the bone or tissue to the E-beam (typically a 5 MeV electron accelerator).

Chemical sterilization procedures are also within the scope of the process of the present invention. More specifically in the case of ethylene oxide, the bone or tissue is, for example, placed under vacuum while 650 mg./liter of ethylene oxide is introduced into the chamber for about 1 to 2 hours at a temperature of e.g.$32^\circ$ C - $57^\circ$ C * after which the gas is removed by vacuum. Similarly, formaldehyde, chlorine dioxide or ozone could be employed as the sterilization gas in place of the ethylene oxide. Vapor phase hydrogen peroxide may also be employed as the sterilant herein. In this case, the bone or tissue is placed in a vacuum chamber and, for example, a 30% $H_2O_2$ liquid solution is vaporized to gas as it enters the chamber at $32^\circ$ C - $57^\circ$ C * whereby the gas decomposes and the vacuum dissipates. Another chemical sterilization technique within the scope of the present invention includes soaking the bone or tissue in ethanol e.g. 70% ethanol, while the bone or tissue is maintained at a low temperature. Other sterilization treatments include soaking in aqueous solutions of quaternary agents, phenolic agents, glutaraldehyde and the like.

In the embodiment of the present invention wherein irradiation is utilized as the sterilization means, it is preferred that the bone or tissue is maintained at a temperature which is low enough to prevent denaturization. Typically these temperatures are below 40°C and are preferably 0°C or

below. Most preferably, irradiation sterilization in accordance with the present invention is carried out on bone or tissue which is maintained in a frozen state, e.g. packed in dry-ice during the sterilization process.

Subsequent to sterilization as described hereinbefore, the resultant sterilized bone or tissue is now aseptically cleansed whereby the bone or tissue is processed, inter alia , to substantially free the bone or tissue of dead bacteria and other microorganisms, as well as pyrogens. It is to be understood herein that for the purposes of the present invention and in accordance with the present invention, aseptic cleansing subsequent to sterilization of the bone or tissue, is defined as including processing such as rinsing the sterilized bone and tissue with water, preferably sterile water for injection, debridement to remove soft tissue and attachments and further processing such as shaping. Optionally, if desired, the debrided bone or tissue may be defatted and/or demineralized and/or milled or ground into chips or bone powder. Defatting is commonly carried out by contacting (e.g. soaking) the bone or tissue with a defatting solution. Conventional defatting solutions include lipid solvents such as alcohols. Following defatting, the bone may be demineralized, if desired. Demineralization is commonly carried out by soaking in acid solution in which the necessary hydrogen ion concentration is maintained while agitating the acid.

Subsequently, all allograft bone and tissue is measured or weighed, final sterility tested and packaged. Processed allograft bone and tissue is typically preserved by freezing or by freeze drying.

Various types of allograft bone and musculoskeletal tissue can be processed in accordance with the present invention including cortical and/or cancellous bone tissue, bone and tendon, ribs, tibia, femur, humerus, fascia lata and the like.

Preferably, the aseptic cleansing step of the present method is carried out in cleanrooms. A cleanroom is an enclosure specially designed to maintain an aseptic environment. More particularly, the aseptic allograft processing for purposes of the present invention is preferably carried out in a class 10 cleanroom. The class number indicate the number of airborne particulates in a size range of 0.3 microns or larger that are allowed per cubic foot. Thus a Class 10 cleanroom allows no more than 10 particles per cubic foot of air. The air is of such high quality that no viable microorganisms are allowed. The cleanroom temperature is preferably maintained at about $18^\circ$ C * and the humidity is

* 90°F  - 135°F

*65°F

preferably maintained at about 40%. Sterilization in accordance with the present invention need not be carried out in a cleanroom, as defined above, although may be if desired.

**Claims**

1. A method for processing allograft bone or musculoskeletal tissue, said method comprising the steps of:

(i) subjecting allograft bone or musculoskeletal tissue to sterilization, said sterilization being sufficient to kill microorganisms but insufficient to cause substantial physical, chemical or biological deterioration of the allograft bone or musculoskeletal tissue; and

(ii) aseptically cleansing the sterilized allograft bone or musculoskeletal tissue resulting from step (i).

2. A method according to claim 1 wherein said sterilization is carried out chemically, thermally or by irradiation.

3. A method according to claim 2 wherein said sterilization is carried out by gamma radiation.

4. A method according to claim 3 wherein said gamma radiation is derived from cobalt-60 gamma ray source.

5. A method according to any one of the preceding claims wherein said aseptic cleansing step (ii) includes rinsing the sterilized allograft bone or musculoskeletal tissue with sterile water.

6. A method according to any one of the preceding claims wherein said aseptic cleansing of step (ii) includes debridement and defatting of the sterilized bone or musculoskeletal tissue.

7. A method according to any one of the preceding claims wherein said aseptic cleansing of step (ii) includes demineralization.